Europäisches Patentamt

**European Patent Office**

Office européen des brevets

(11) Publication number: **0 240 197**
A2

(12) **EUROPEAN PATENT APPLICATION**

(21) Application number: **87302211.5**

(22) Date of filing: **16.03.87**

(51) Int. Cl.⁴: **C07C 69/82** , C07C 69/80 , C07C 67/08

(30) Priority: **17.03.86 US 840822**

(43) Date of publication of application:
**07.10.87 Bulletin 87/41**

(84) Designated Contracting States:
**AT BE CH DE ES FR GB GR IT LI LU NL SE**

(71) Applicant: **CELANESE CORPORATION**
**1211 Avenue of the Americas**
**New York New York 10036(US)**

(72) Inventor: **Alam, Mahfuzul**
**4901 Eider Drive**
**Corpus Christi Texas(US)**
Inventor: **Ranade, G.R.**
**18 Wayne Drive**
**East Lyme Connecticut(US)**

(74) Representative: **De Minvielle-Devaux, Ian**
**Benedict Peter et al**
**CARPMAELS & RANSFORD 43, Bloomsbury**
**Square**
**London WC1A 2RA(GB)**

(54) **Improved reaction time for the manufacture of diphenylisophthalate/diphenylterephthalate mixture.**

(57) A process for the improved reaction time for the formation of a diphenylisophthalate/diphenylterephthalate monomer from phenol, isophthalic acid, and terephthalic acid. The improvement in reaction time is achieved by maintaining a vacuum throughout the reaction system during the entire length of the reaction. This results also in a greater recovery of water from the reaction system as well as a higher degree of esterification.

EP 0 240 197 A2

## IMPROVED REACTION TIME FOR THE MANUFACTURE OF
## DIPHENYLISOPHTHALATE/DIPHENYLTEREPHTHALATE MIXTURE

This invention relates to the synthesis of diphenylisophthalate/diphenylterephthalate monomers by reacting isophthalic acid and terephthalic acid with phenol. The invention involves the removal of water from the reaction system as soon as possible so as to obtain the lowest possible reaction time.

### BACKGROUND OF THE INVENTION

U. S. Patent 3,068,206, issued to Nicolson et al discloses a polyester synthetic reaction system wherein one or more polyhydric alcohols such as ethylene or propylene glycol or glycerol are reacted with one or more polycarboxylic acids or their anhydrides. A modifying agent may or may not be used. The reaction is preferably carried out at temperatures from room temperature to about 300°C. U. S. Patent 3,039,980, issued to Mallison, discloses a polyhydric alcohol/polycarboxylic acid reaction wherein water of the esterification reaction is removed from the reaction zone by passing through the reaction zone an inert, organic, normally liquid solvent. U. S. Patent 3,109,831, issued to Seiner, discloses a reaction between a polyol and a polyester of a dicarboxylic acid whereby the volatilization of the polyol is held to a minimum. U. S. Patent 3,819,585, issued to Funk et al, discloses the formation of a polyester from ethylene glycol and terephthalic acid. The reaction has two phases wherein the first phase is conducted at a pressure of about 0-5 psig and through about 75-95% of esterification, and the second phase is conducted at a substantially higher pressure than the first phase during which the percent of esterification is increased to about 95-99%. U. S. Patent 4,146,729, issued to Goodley et al, discloses an esterification reaction between terephthalic acid and ethylene glycol whereby the excess glycol is recovered from reaction off-gases. U. S. Patent 4,204,070, issued to Suzuki et al, discloses a process for preparing bis(hydroxyethyl)terephthalate where terephthalic acid is dissolved in molten bis(hydroxyethyl)-terephthalate and its low molecular weight oligomers at a temperature above the dew point of ethylene glycol. Ethylene glycol is fed into the solution at a controlled rate so that the ethylene glycol does not distill off. U. S. Patent 4,254,246, issued to Dicoi et al, discloses a reaction of ethylene glycol and terephthalic acid in the presence of a catalyst so that the vapor by-products from the esterification, prepolycondensation and polycondensation stages are continuously rectified yielding high-purity ethylene glycol (99.85%) which is returned to the process.

U. S. Patent 3,418,286, issued to Schmidt et al, discloses the reaction of polyhydric alcohol and a carboxylic acid to produce a polyester resin by a fusion process. The reaction can be carried out at temperatures above 350°F. A vacuum or reduced pressure is also applied to the system, but not until the reaction has gone 1/3 of the way to completion and before the reaction has gone 3/4 of the way to completion.

### SUMMARY OF THE INVENTION

This invention relates to the shortening of the reaction time for the synthesis of diphenylisophthalate/diphenylterephthalate monomers by removing water formed in the reaction as quickly as possible so as to decrease the reversible chemical reaction. The maximum amount of water is removed and the amount of phenol lost from the reaction mixture is minimized.

### DETAILED DESCRIPTION OF THE INVENTION

In the synthesis of diphenylisophthalate/diphenylterephthalate monomers by reaction of isophthalic acid and terephthalic acid with phenol, the problem of water removal from the reaction mixture can be looked upon as being composed of two steps. The first step is the removal of water vapor from the liquid reaction mixture to the vapor phase in the system. The second step is to condense the vapor obtained from the reactor using a column and condenser combination.

It is observed that the evacuation of the system comprising reactor, column, and condenser after the initial charging of reactants results in a significant increase in the distillate collection rates. The increase in the distillate collection rate is so substantial that a high distillate collection rate can be maintained, even at high reflux ratios ranging, for example, from 10:1 to 30:1. It is also observed that the higher the amount of vacuum the better the distillate collection rate, thus leading to an improved reaction time. Higher reflux ratios enrich the distillate, the collection of which is the only source of water removal from the system.

The following examples will demonstrate the improved reaction time and degree of esterification by the process of this invention.

Example 1

A monomer reaction was carried out by charging a reactor as follows:
Phenol 166 lbs.
Isophthalic acid 65.75 lbs.
Terephthalic acid 8.5 lbs.
$Sb_2O_3$(2 mole %) 1166 grams

The reaction was carried out at a final temperature of 285°C. The amount of distillate collected after the entire run of 12 hours was a 47 lb. mixture of water and phenol. The degree of esterification was 92%. No evacuation was carried out after the initial charge of the mixture.

Example 2

A monomer reactor was charged as follows:
Phenol 170 lbs.
Isophthalic acid 37.5 lbs.
Terephthalic acid 12.5 lbs.
$Sb_2O_3$ 788 grams

A vacuum of 25° was pulled after the initial charge. The total reaction time was 6 hours, during which a 72 lb. mixture of water and phenol was collected. The degree of esterification was 98%.

The above examples show that by applying a vacuum to the reaction system throughout the entire reaction, the time for the completion of the reaction is decisively shortened, the degree of esterification is increased, and there is a greater recovery of water from the reaction system.

**Claims**

1. In a process for the formation of diphenylisophthalate/diphenylterephthalate monomer from a reaction mixture comprising isophthalic acid, terephthalic acid, and phenol, the improvement which consists essentially of maintaining a vacuum throughout the reaction system during the entire length of the reaction, thereby obtaining an increased recovery of water from the reaction system and a higher degree of esterification of the reactants.

2. The process in accordance with claim 1 wherein the vacuum is maintained throughout the system during the entire length of the reaction at a pressure of about 25 inches.